# EUROPEAN PATENT APPLICATION

(11) **EP 2 939 598 A1**
(43) Date of publication of application: **04.11.2015**
(21) Application number: 13869310.6
(22) Date of filing: 04.12.2013
(51) Int. Cl.: A61B 8/00

(54) **ULTRASONIC DIAGNOSTIC DEVICE AND ELASTICITY EVALUATION METHOD**

(30) Priority: 25.12.2012 JP 2012280750
(71) Applicant: Hitachi Aloka Medical, Ltd., Tokyo 181-8622 (JP)
(72) Inventor: YOSHIKAWA, Hideki, Tokyo 100-8280 (JP); ASAMI, Rei, Tokyo 100-8280 (JP); TABARU, Marie, Tokyo 100-8280 (JP)
(74) Representative: MERH-IP Matias Erny Reichl Hoffmann
(86) International application number: PCT/JP2013/082607
(87) International publication number: WO 2014/103642

(57) **Abstract**

Provided is an ultrasonic diagnostic apparatus configured to perform speed measurement while lessening the influence of the wave surface feature and scattering resulting from the shear wave propagation, which transmits burst wave as first ultrasonic wave to the subject from the probe 11 and ultrasonic transmission-reception section 13 to apply radiation pressure. The track pulse wave as the second ultrasonic wave is transmitted to and received from the subject to detect displacement of medium in the subject resulting from the shear wave propagation in the subject at the radiation pressure. The elasticity evaluation section 17 of the controller 12 measures the first arrival time at the first depth and the second arrival time at the second depth of the shear wave with the single track pulse wave at the predetermined angle θ(≠0) to the subject depth direction using the data received from the ultrasonic transmission-reception section 13. The display section 15 displays the elasticity information of the subject by calculating the shear wave propagation velocity based on the difference between the first and the second arrival time values.

## Description

### Technical Field

The present invention relates to an ultrasonic diagnostic technique which generates a shear wave in the subject by means of acoustic radiation force so that elasticity of the subject is evaluated based on the propagation velocity of the shear wave.

### Background Art

The technique for evaluating elastic modulus of the tissue by means of the transversal wave (hereinafter referred to as shear wave) has been attracting attention for application to the image display device using ultrasonic waves. An attempt to apply such technique to the clinical use for mammary tumor and chronic liver disease has been carried on. This approach generates the shear wave within the tissue as the measuring object, and evaluates the generated shear wave by means of ultrasonic waves so as to calculate physical quantity with respect to speed or rigidity. When it is assumed that the compressional wave velocity is sufficiently higher than the transverse wave velocity through conversion by setting Poisson's ratio of the tissue to 0.5, the Young's modulus may be simply expressed by a formula of E=3ρVs² (E: Young's modulus, p: density, Vₛ: shear wave velocity).

The radiation force method as one of approaches to generate the shear wave applies radiation pressure to the inside of the living body through the focused ultrasonic wave that converges the ultrasonic waves to a local area in the tissue so as to generate the shear wave by a resultant tissue displacement. Patent Literature 1 discloses the related art concerning the radiation force method. For the purpose of estimating the shear wave with higher accuracy, the disclosed radiation force method is intended to obtain the correlation among displacement profiles derived from a plurality of different combinations of the source position and the detection position, and to detect the shear wave information using the displacement resulting from various spatial combinations of the transmission position and the detection position so that the shear wave velocity is calculated at the respective lateral positions.

### Citation List

### Patent Literature

[Patent Literature 1] JP-A-2012-81269

### Summary of the Invention

### Technical Problem

The shear wave which propagates through the tissue exhibits complicated features depending on viscosity of medium, anisotropy of the structure, and existence of the structure which contributes to scattering. For example, if the blood vessel exists on the propagation path, the surface of the arriving wave is disturbed under the influence of scattering and diffraction, resulting in reduced speed measurement accuracy. Therefore, measurement of the shear wave velocity requires the technique capable of lessening the disturbance of wave surface by reducing the propagation distance of the wave surface, which is required for the speed measurement as short as possible, and ensuring the stable measurement accuracy even if the wave surface disturbance occurs.

It is an object of the present invention to provide a highly accurate ultrasonic diagnostic apparatus and an elasticity evaluation method for lessening the influence of the wave surface disturbance. Solution to Problem

The present invention provides an ultrasonic diagnostic apparatus which includes a probe which transmits and receives ultrasonic waves to and from a subject, and a control unit which generates a shear wave in the subject by transmitting a first ultrasonic wave to the subject via the probe, and allows transmission and reception of a second ultrasonic wave to and from the subject. The control unit includes a speed measurement section for measuring a first arrival time and a second arrival time of the shear wave generated in the subject based on the second ultrasonic wave, and measuring propagation velocity of the shear wave based on a difference between the first arrival time and the second arrival time.

The present invention further provides an elasticity evaluation method for evaluating elasticity of a subject by generating a shear wave in the subject to which a first ultrasonic wave is transmitted from a probe, transmitting and receiving a second ultrasonic wave to and from the subject, measuring a first arrival time and a second arrival time of the shear wave generated in the subject based on the second ultrasonic wave, and measuring a propagation velocity of the shear wave based on a difference between the first arrival time and the second arrival time.

### Advantageous Effects of Invention

The present invention provides the ultrasonic diagnostic apparatus with high measurement accuracy and the elasticity evaluation method for lessening the influence of the wave surface disturbance.

### Brief Description of the Drawings

[Figure 1]

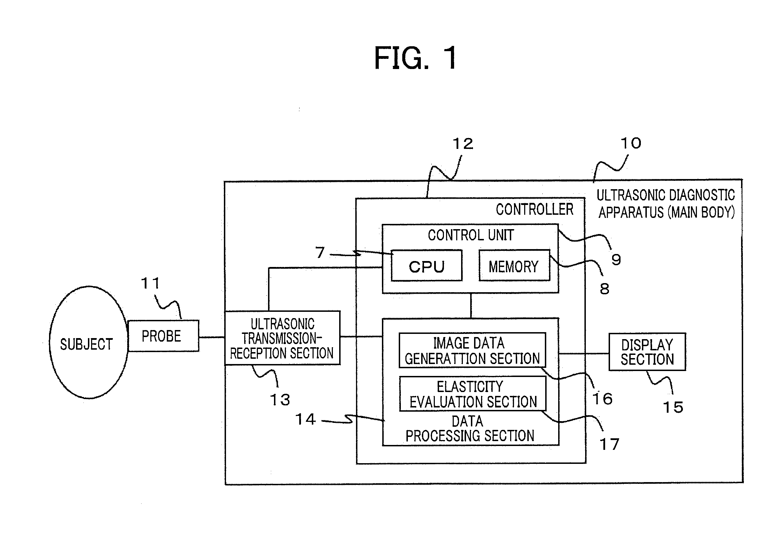

   FIG. 1 is a block diagram showing an exemplary structure of an ultrasonic diagnostic apparatus according to a first embodiment.
[Figure 2]

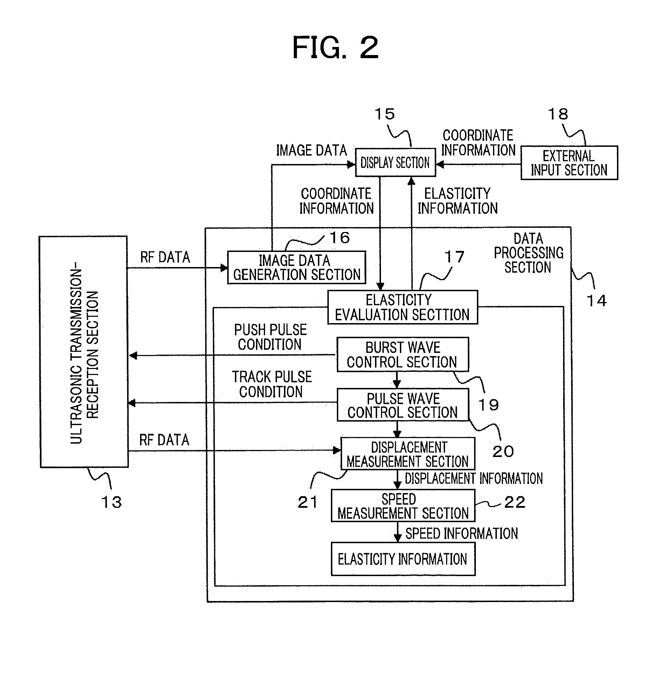

   FIG. 2 is a functional explanatory view of the ultrasonic diagnostic apparatus according to the first embodiment.
[Figure 3]

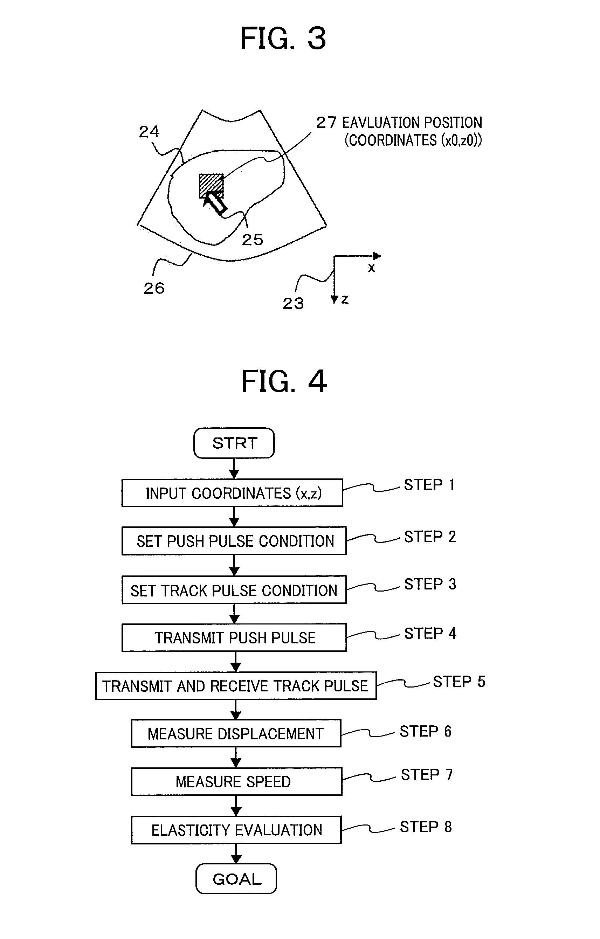

   FIG. 3 is an explanatory view representing a method of setting an evaluation position according to the first embodiment.
[Figure 4]
   FIG. 4 is a flowchart of process steps for an elasticity evaluation method according to the first embodiment.
[Figure 5]

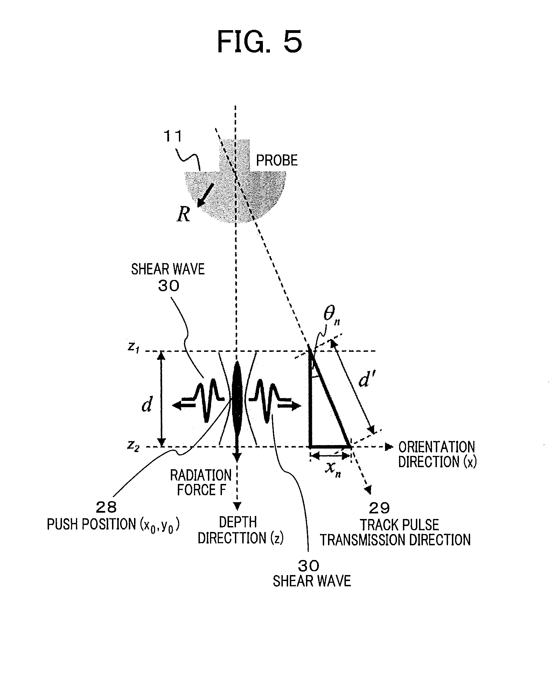

   FIG. 5 is an explanatory view representing a position to which a track pulse is transmitted according to the first embodiment.
[Figure 6]

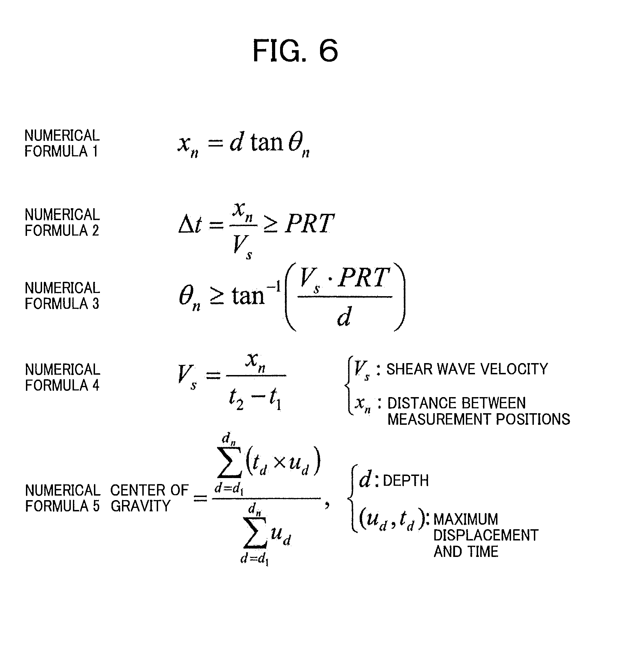

   FIG. 6 shows numerical formulae of the respective embodiments.
[Figure 7]

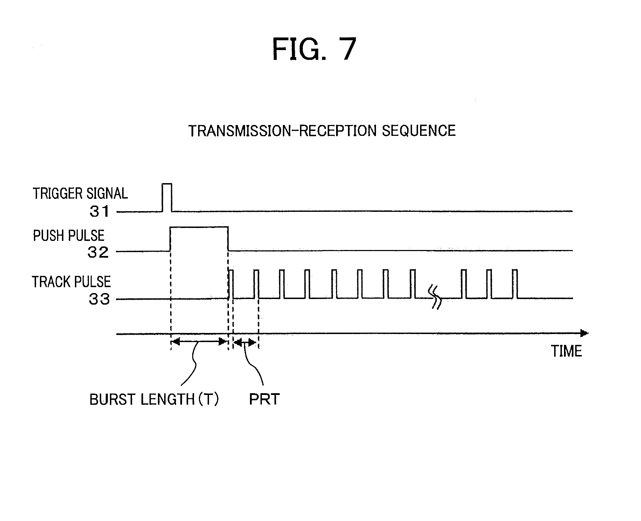

   FIG. 7 is a view illustrating a transmission sequence according to the first embodiment.
[Figure 8]

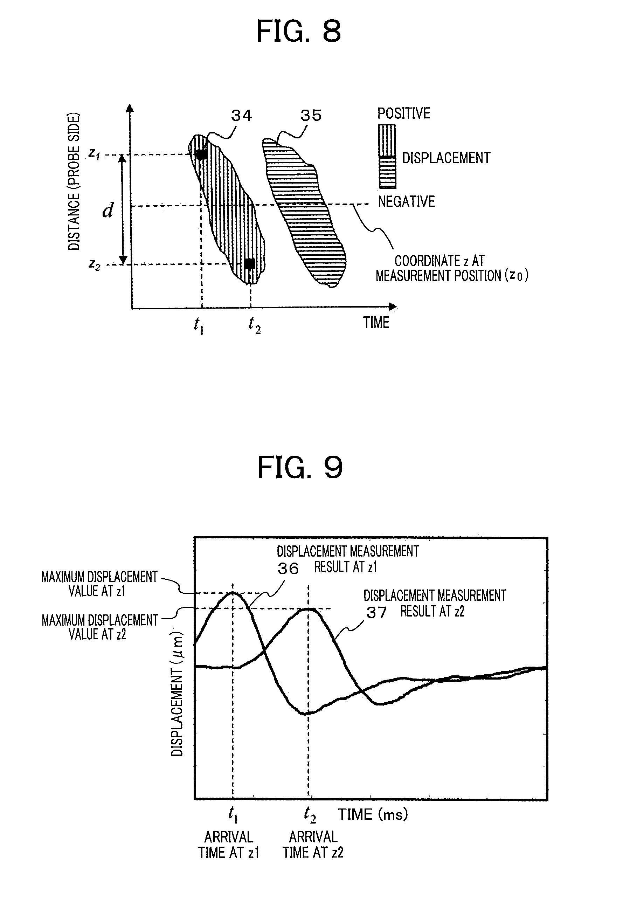

   FIG. 8 is a view illustrating a displacement measurement result according to the first embodiment.
[Figure 9]
   FIG. 9 is an explanatory view representing a method of measuring the arrival time according to the first embodiment.
[Figure 10]

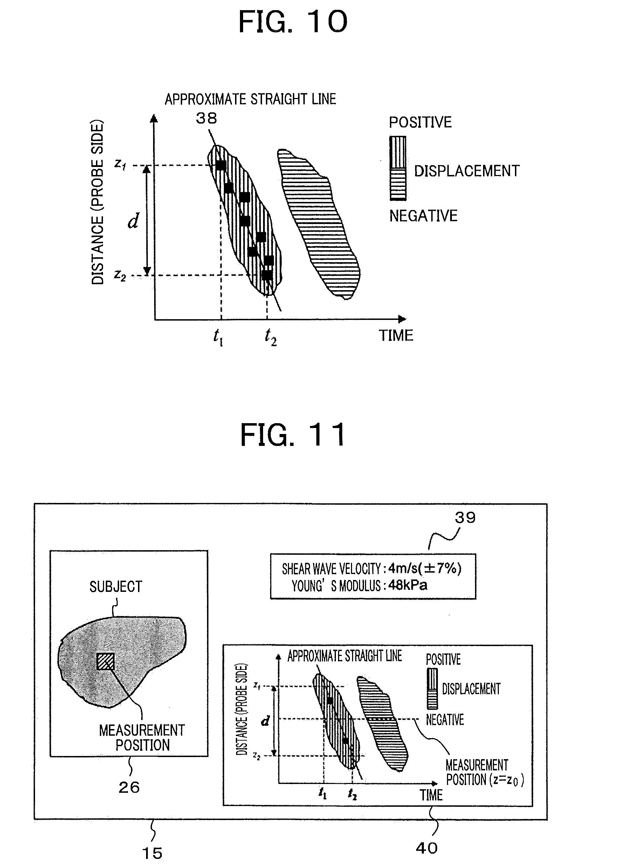

   FIG. 10 is an explanatory view representing a method of calculating the arrival time based on the approximate straight line according to the first embodiment.
[Figure 11]
   FIG. 11 illustrates a display example of a display section according to the first embodiment.
[Figure 12]

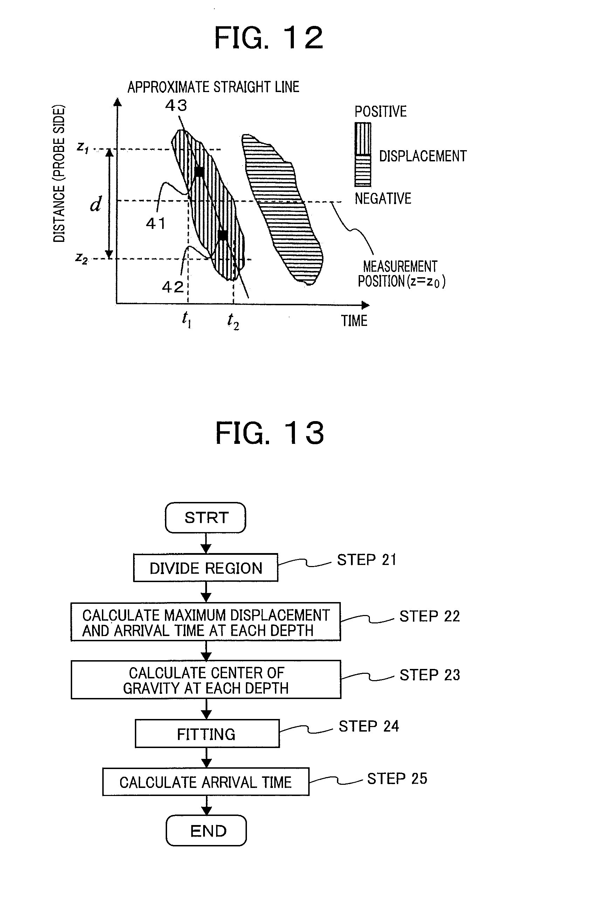

   FIG. 12 is an explanatory view representing a method of calculating the arrival time according to a second embodiment.
[Figure 13]
   FIG. 13 is a flowchart of process steps for calculating the center of gravity as a feature point indicating a region according to the second embodiment.
[Figure 14]

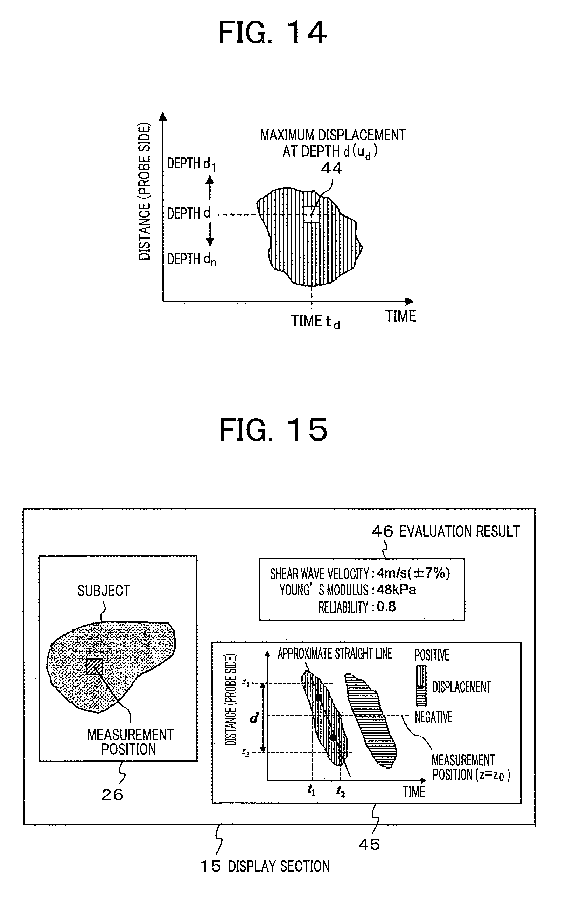

   FIG. 14 is an explanatory view representing calculation of the center of gravity as the feature point indicating the region according to the second embodiment;
[Figure 15]
   FIG. 15 illustrates a display example of the display section according to the second embodiment.
[Figure 16]

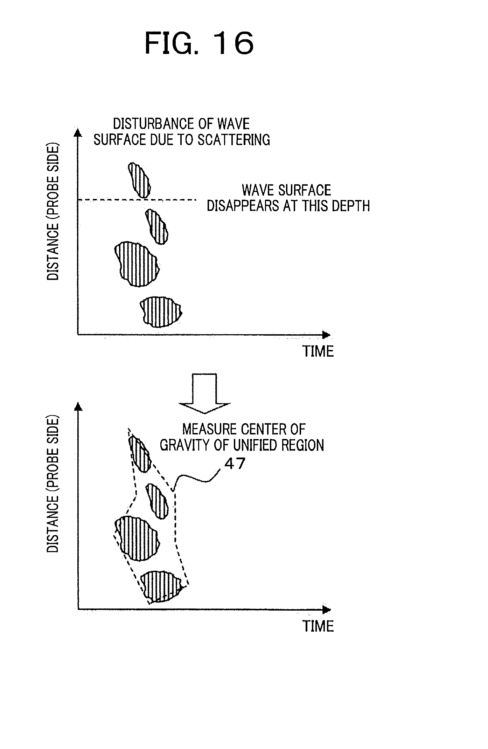

   FIG. 16 is an explanatory view representing the effect obtained by using the center of gravity as the feature point indicating the region according to the second embodiment.

### Description of Embodiments

Embodiments of the present invention will be described referring to the drawings. In the specification, the "elasticity information" of the tissue is defined as the physical property in general concerning deformation and flow of the substance, for example, a strain, a shear wave velocity, a compressional wave velocity, a Young's modulus, a modulus of rigidity, volume elastic modulus, a Poisson's ratio, coefficient of viscosity and the like.

### [First Embodiment]

The ultrasonic diagnostic apparatus and the elasticity evaluation method according to the first embodiment will be described referring to the block diagram shown in FIG. 1, the functional explanatory view shown in FIG. 2, the explanatory view representing the method of setting the evaluation position shown in FIG. 3, a flowchart of process steps for the elasticity evaluation method shown in FIG. 4, and an explanatory view representing the position to which the track pulse is transmitted shown in FIG. 5. The ultrasonic diagnostic apparatus according to this embodiment includes a probe 11 for transmission and reception of the ultrasonic waves to and from the subject, and a controller 12 which generates the shear wave in the subject through transmission of a first ultrasonic wave to the subject via the probe 11, and further transmits and receives a second ultrasonic wave to and from the subject. The controller 12 includes a speed measurement section 22 for measuring the propagation velocity of the shear wave based on the difference between the first and the second arrival time values of the shear wave generated in the subject, which have been measured based on the second ultrasonic wave.

This embodiment also describes the method of evaluating elasticity of the subject, which includes the process steps of generating the shear wave in the subject by transmitting the first ultrasonic wave from the probe 11 to the subject, measuring the first and the second arrival time values of the shear wave generated in the subject based on the second ultrasonic wave received and transmitted from and to the subject, and measuring the propagation velocity of the shear wave based on the difference between the first and the second arrival time values.

Although the probe to be used is not specifically limited, the embodiment will be described on the assumption that the convex type probe is used, having the bore part curved to form a convex shape at the side of the living body.

The structure of an ultrasonic diagnostic apparatus configured to generate high frequency (RF) data and image data, which is employed for the embodiment will be described referring to FIG. 1. As FIG. 1 shows, the ultrasonic diagnostic apparatus 10 includes the controller 12, an ultrasonic transmission-reception section 13, and a display section 15. The controller 12 has a control unit 9 which includes a central processing unit (CPU) 7 as the processing section and a memory 8 as a storage unit, and a data processing section 14. An electric signal for transmission pulse is generated by the ultrasonic transmission-reception section 13 under the control of the control unit 9 of the controller 12. The electric signal for transmission pulse generated by the ultrasonic transmission-reception section 13 is converted into an analog signal through a D/A converter so as to be sent to the probe 11 grounded to the surface of the subject such as the living body. The electric signal input to the probe 11 is converted into an acoustic signal through an internally installed ceramic element, and transmitted into the subject. The transmission is carried out by a plurality of ceramic elements, each of which has a predetermined time delay added to converge at a predetermined depth in the subject.

The acoustic signal reflected in the propagation process in the subject is received by the probe 11 again, and converted into the electric signal reverse to the transmission process. It is then sent to the A/D converter by a selector switch inside the ultrasonic transmission-reception section 13 so as to be converted into a digital signal. The ultrasonic transmission-reception section 13 is configured to subject the signals received by the respective elements to the addition process such as the phasing addition in consideration of the time delay added to the respective elements in the transmission process. The signal is further subjected to the process such as attenuation correction, and sent to the data processing section 14 in the controller 12 as the complex RF data.

The data processing section 14 includes an image data generation section 16 which can be implemented by executing the predetermined program and an elasticity evaluation section 17 to be described later in detail referring to FIG. 2. The RF data acquired by the data processing section 14 from the ultrasonic transmission-reception section 13 become specific one-line element data along the ultrasonic wave transmission-reception direction among the eventually displayed image data. Transmission and reception of ultrasonic waves to and from the subject are switched sequentially in the row direction of the ceramic elements that constitute the probe 11 so that all the RF data that constitute the two-dimensional image data are acquired and stored in the memory.

The thus acquired and stored RF data are converted into two-dimensional image data by the image data generation section 16 of the data processing section 14. Specifically, the aforementioned process is a generally employed image generation process, for example, gain control, logarithmic compression, envelope demodulation, scan conversion or the like, which has been performed by the commonly available ultrasonic diagnostic apparatus. The image data generated by the image data generation section 16 are displayed on the display section 15. The data processing section 14 of the control unit 12 may be configured by the CPU as the processing unit and the memory that stores the program and data. It is also possible to use the CPU 7 and the memory 8 of the aforementioned control unit 9 as those for the data processing section.

The elasticity evaluation function performed by the elasticity evaluation section 17 of the data processing section 14 according to the embodiment will be described referring to the functional explanatory view of FIG. 2, the schematic view of FIG. 3, and the flowchart of FIG. 4.

Referring to FIG. 2, the same elements as those shown in FIG. 1 will be designated with the same codes. An external input section 18 is configured to allow the operator to input coordinate information about the evaluation position. The data processing section 14 implements the elasticity evaluation section 17 under the control of the controller 12 or by itself. The elasticity evaluation section 17 generates the radiation pressure at the evaluation position designated by the operator so that the elasticity is evaluated by means of the propagating shear wave. As the drawing shows, the elasticity evaluation section 17 includes a burst wave control section 19 and a pulse wave control section 20 for setting the push pulse condition and the track pulse condition, respectively to the ultrasonic transmission-reception section 13. The section further includes a displacement measurement section 21 for generating the displacement information by means of the RF data acquired from the ultrasonic transmission-reception section 13, and the speed measurement section 22 for generating the speed information from the displacement information. The elasticity information is provided based on the thus acquired speed information as a result of the elasticity evaluation.

The operator designates the evaluation position by inputting the coordinate information to the image data including the subject displayed on the display section 15 through the external input section 18 provided for the main body of the apparatus, for example, the mouse and the trackball. Referring to the schematic view shown in FIG. 3, the operator designates the coordinate information on the desired position of the subject 24 in the image data 26 with a pointer 25 so as to set the coordinates (x₀,z₀) of the evaluation position 27 in the image data 26. It is assumed to use a rectangular coordinate system 23, in which the x denotes the transverse direction, and y denotes the longitudinal direction. As described above, the elasticity information is derived from propagation of the shear wave. In other words, a certain region is required for the elasticity evaluation. Accordingly, an evaluation position 27 (x₀,z₀) corresponds to the coordinates of a push position 28 for generating the radiation force as the origin position from which the shear wave is propagated.

The set coordinate information is sent to the data processing section 14, and input to the elasticity evaluation section 17 installed therein in step 1 of FIG. 4. Then in step 2, the burst wave control section 19 sets the wave transmission condition of the first ultrasonic wave, that is, the burst wave as the push pulse based on the input coordinate data. The wave transmission condition which hardly influences the living body and allows effective generation of the shear wave may be set as the conversion condition suitably in the range of the F number set to 1 to 2 (the value obtained by dividing the bore width by the focal depth), the intensity set to 0.1 to 1 kW/cm², and the burst length set to 100 to 1000 µs. The bore width is in the range of the ceramic element that is actually driven, taking a discrete value at an element interval. In order to form the ideal focus region, each voltage applied to the respective elements is multiplied by the bore weighting (apodization). The push pulse is transmitted along the depth direction of the subject, and accordingly, the transmission angle as the transmission condition becomes zero.

Preferably, the disturbance of the focus region under the influence of diffraction is suppressed by reducing the weighting from the bore center to the corner. The bore weighting is disadvantageous in reducing the intensity. If the evaluation position is located in the deep part and likely to be easily influenced by attenuation, the intensity is prioritized to formation of the region to reduce the bore weighting. It is effective to set the transmission frequency to be approximate to the center frequency of the sensitivity band of the probe 11. The wave transmission condition of the burst wave as the push pulse, which functions as the first ultrasonic wave is immediately sent to the ultrasonic transmission-reception section 13, and applied into the living body via the probe 11.

In step 3, the pulse wave control section 20 sets the wave transmission condition of the second ultrasonic wave, that is, the track pulse. The acoustic parameters such as the frequency, wave number, and F number are substantially the same as those for generating the image data. If the abdomen is set as the inspection object, the condition having the frequency from 1 to 5 MHz, the wave number from 1 to 3, and the F number in the range from 1 to 2 may be used. As described above, the track pulse functioning as the second ultrasonic wave is the pulse wave to be transmitted and received for the purpose of measuring the displacement of the tissue resulting from propagation of the shear wave. The shear wave is sharply attenuated as it is propagated. Therefore, the transmission direction and the transmission angle of the second ultrasonic wave, that is, track pulse become essential wave transmission conditions. The pulse wave control section 20 according to the embodiment sets the transmission direction of the track pulse based on the shear wave velocity expected as the coordinate information for realizing the elasticity evaluation. The pulse wave control section 20 according to this embodiment transmits the track pulse only in the specific transmission direction, in other words, only one measurement position is set.

Referring to FIG. 5, an explanation will be made with respect to the method of setting each transmission direction and transmission angle of the push pulse as the first ultrasonic wave and the track pulse as the second ultrasonic wave. FIG. 5 schematically illustrates the probe 11 with curvature of R, the radiation force F generated at the push position 28 (x₀,z₀), and a shear wave 30 propagated from the push position in the orientation direction. It is assumed that the width of the depth direction on which the radiation force F acts is set to d(z₁ to z₂), the angle formed by the transmission direction of the track pulse as the second ultrasonic wave and the depth direction as the center axis of the probe 11, in other words, the application direction of the radiation force F is set to θₙ(θₙ≠0), and the base and the oblique side of the right angled triangle formed in the range of the width d in the depth direction as shown in the drawing is set to xₙ and d', respectively. A transmission direction 29 of the track pulse is discretely switched for each width of the elements that constitute the probe. The subscript n denotes the center position of the drive element, that is, the transmission direction of the track pulse. The drawing shows that the xₙ is expressed as tangent of the numerical formula 1 of FIG. 6 using θₙ as the angle information. As described above, the push pulse condition and the track pulse condition are input to the ultrasonic transmission-reception section 13 from the burst wave control section 19 and the pulse wave control section 20 of the elasticity evaluation section 17, respectively. The angle θₙ in the transmission direction is not set to zero, which represents that the controller 12 controls the transmission direction of the second ultrasonic wave to be different from that of the first ultrasonic wave.

The shear wave 30 generated by the radiation force of the push pulse as the first ultrasonic wave having the push pulse condition set by the burst wave control section 19 as shown in FIG. 2 is propagated to form substantially a cylindrical wave with respect to the push pulse transmission direction as the axis while displacing the tissue in the depth direction (z direction). The propagation range in the depth direction becomes d on which the radiation force substantially acts. If the displacement measurement section 21 performs the displacement measurement in the transmission direction of the track pulse having the track pulse condition set by the pulse wave control section 20 by means of the RF data from the ultrasonic transmission-reception section 13, the shear wave 30 propagated from the push position is first measured at the depth z₁, and then measured at the depth z₂. Each value of the first and the second arrival times of the shear wave 30 at the respective depths z₁ and z₂ is measured so as to allow the speed measurement section 22 to calculate the shear wave velocity Vₛ using the time difference □t and the distance xₙ.

The controller 12 controls so that the depth in the subject, at which the first arrival time is measured differs from the depth at which the second arrival time is measured. The controller 12 also controls so that the depth in the subject where the second arrival time is measured is larger than the depth in the subject where the first arrival time is measured.

The pulse groups that constitute the track pulse are transmitted and received by the ultrasonic transmission-reception section 13 in a constant pulse reputation time (PRT). The PRT becomes the time resolution in the displacement measurement. Accordingly, it is necessary to satisfy the condition of the numerical formula 2 shown in FIG. 6 when measuring the time difference □t. This formula also represents the condition to be satisfied by the xₙ. It is necessary for performing the measurement in preliminary consideration of the speed range to be measured since the Vₛ is the shear wave velocity of the tissue. For example, in evaluating fibrillation of the liver tissue, the Vₛ may be set to be in the range from approximately 1 to 5 m/s. In evaluating the breast cancer, the velocity may be set to be further higher, and accordingly, the maximum speed is expected to be set to 10 m/s.

From the numerical formulae 1 and 2 shown in FIG. 6, the transmission direction of the track pulse and the transmission angle θₙ are set to satisfy the condition of the numerical formula 3 shown in FIG. 6. As the shear wave is influenced by the attenuation and scattering in the propagation process, the minimum value that satisfies the numerical formula 3 is set for the purpose of minimizing the aforementioned influence. If cirrhosis of the liver is assumed to be positioned at the depth of 5 cm (Vₛ=4m/s), and it is measured with the push pulse (d=10mm) with the F number set to 1, and the track pulse with PRT=0.25 ms, the transmission direction of the track pulse of θₙ=0.1 rad is obtained. The track pulse which includes a series of pulse groups is transmitted in the predetermined direction θₙ(θₙ≠0) from the ultrasonic transmission-reception section 13 via the probe 11 shown in FIG. 1 based on the track pulse condition equal to or higher than the one set by the pulse wave control section 20 (step 5).

FIG. 7 shows an example of the transmission sequence of the push pulse as the first ultrasonic wave and the track pulse as the second ultrasonic wave, which are set by the burst wave control section 19 and the pulse wave control section 20 of the elasticity evaluation section 17 of the apparatus according to the embodiment. Upon input of the coordinates of the evaluation position in step 1, a trigger signal 31 as an electric signal is immediately sent to the data processing section 14 from the control unit 9 of the controller 12. Simultaneously with the input of the trigger signal 31, the push pulse condition of the first ultrasonic wave, and the track pulse condition of the second ultrasonic wave are determined by the burst wave control section 19 and the pulse wave control section 20 of the elasticity evaluation section 17. Based on the aforementioned conditions, a push pulse 32 with the burst length T as the first ultrasonic wave is transmitted from the ultrasonic transmission-reception section 13. Subsequently, a series of pulse groups that constitute the track pulse 33 as the second ultrasonic wave is transmitted and received at the predetermined PRT. Transmission and reception of the track pulse 33 may be started simultaneously with the input of the trigger signal 31, that is, the push pulse 32. In this case, as noise may be received during application of the push pulse 32, it is therefore difficult to measure the displacement. However, this approach is advantageous to ensure the RF data before and after the application to be acquired.

As FIG. 7 shows, through the series of sequence including input of the trigger signal 31, one transmission of the push pulse 32 as the first ultrasonic wave, and transmission and reception of the track pulse 33 formed of a plurality of pulse groups, the displacement measurement section 21 shown in FIG. 2 provides the displacement information, and the speed measurement section 22 calculates the measurement result of the shear wave velocity from the displacement information. Repetition of the series of sequence including the trigger signal 31, the push pulse 32 and the track pulse 33 shown in FIG. 7 a plurality of times provides a plurality of measurement results. The statistics such as the standard deviation of the measurement results are calculated and displayed on the display section 15 as one of data indicating the measurement accuracy. The accuracy will be described as an example later in the explanation referring to FIG. 11.

A reflection signal from the living body acquired through transmission of the track pulse 33 is sent to the ultrasonic transmission-reception section 13 via the probe 11 so as to generate a plurality of complex RF data as described above. As FIG. 2 shows, the RF data are input to the displacement measurement section 21 of the data processing section 14 so that the tissue displacement resultant from the shear wave propagation is measured in step 6 of FIG. 4, and the displacement information is provided. The displacement measurement function of the displacement measurement section 21 is implemented by performing complex correlation calculation between the RF data acquired at the time interval of PRT. In this case, the particle velocity is calculated as the displacement per unit of time. There may be the method of calculating the absolute value of the displacement in reference to the RF data before transmission of the push pulse. The particle velocity serves to remove the low frequency component resulting from deflection of the probe and natural movement of the biotissue so as to be effective for measuring the shear wave with high sensitivity. In this embodiment, therefore, the displacement measurement section 21 acquires the displacement information using the particle velocity. The displacement measurement section 21 sets the spatial range for performing the displacement measurement based on the push pulse transmission condition determined by the burst wave control section 19, and sets the time range based on the track pulse transmission condition determined by the pulse wave control section 20.

All the acquired RF data are subjected to the calculation, and the shear wave velocity is measured by the speed measurement section 22 in step 7 based on the calculated displacement information so as to determine the speed information. In step 8, based on the speed information determined by the speed measurement section 22, the elasticity information is acquired as the elasticity evaluation result.

FIG. 8 shows an example of the displacement data and the displacement information derived from the displacement measurement section 21, that is, an example of a displacement 34 at positive side and a displacement 35 at negative side of the displacement information as a result of the displacement measurement in the track pulse transmission direction. Referring to the drawing, y-axis denotes the distance in the depth direction, and x-axis denotes the time. The positive displacement 34 which appears precedingly in time shows the arrival time delay from the upper end (coordinate: z₁, arrival time: t₁) to the lower end (coordinate: z₂, arrival time: t₂) of the right angled triangle as shown in FIG. 5. In this embodiment, the displacement measurement is carried out along the track pulse transmission direction. Accordingly, the distance between z₁ and z₂ at the y-axis of the graph shown in FIG. 8 is set to d. The coordinates (z₁,z₂) of the upper end and the lower end are obtained as z₁=z₀-d/2 and z₂=z₀+d/2 based on the push position and the propagation range d. In this embodiment, the arrival time values of the shear wave at the depths of z₁ and z₂ are calculated through the method using the maximum value or the minimum value of the displacement.

FIG. 9 shows displacement measurement results 36, 37 each as the one-dimensional profile of the displacement data at the depths of z₁ and z₂ shown in FIG. 8. The time taken for reaching the maximum value of the respective displacement profiles while focusing the positive displacement 34 is measured to calculate the arrival time values (t₁,t₂) of the shear wave at the respective depths. An arbitrary index may be used as the feature amount of the displacement profile for calculating the arrival time values (t₁,t₂), for example, the minimum value, the median value between the maximum and the minimum values besides the maximum value so long as it is uniquely set from the displacement profile. The shear wave velocity Vₛ is calculated from the measured arrival time values (t₁,t₂) and the distance (xₙ) in the orientation direction based on the numerical formula 4 shown in FIG. 6.

FIG. 10 represents another method of calculating the arrival time values (t₁, t₂) from the displacement data and displacement information. A plurality of measurement points may be set in the range (z₁-z₂) used for the measurement, for example, the maximum value is used for calculating the arrival time values at the respective measurement points. The use of combinations of calculated values of the distance and the arrival time are used to calculate the one-dimensional approximate straight line as shown in FIG. 10. Intersection points between the approximate straight line, and the z=z₁ and z=z₂ may be employed to calculate the arrival time values (t₁,t₂).

As described above, this embodiment is configured to evaluate the tissue elasticity information in the last step 8 shown in FIG. 4. The tissue elasticity information is defined as the physical property in general concerning deformation and flow of the substance, for example, strain, a shear wave velocity, compressional wave velocity, a Young's modulus, modulus of rigidity, volume elastic modulus, Poisson's ratio, coefficient of viscosity and the like. In the first embodiment, the shear wave velocity has been described in detail as the tissue elasticity information. However, it is possible to convert the measured displacement into the strain, or to conduct frequency analysis of the shear wave to provide such information as viscosity.

FIG. 11 shows a display example of the display section 15 according to the embodiment. The display section 15 is employed to display the image data or propagation velocity of the shear wave, and the displacement data as the displacement information and the center of gravity to be described later. In other words, the evaluated elasticity information is sent to the display section 15, and presented to the operator together with the image data 26 described referring to FIG. 3 and the displacement shown in FIG. 8 as the evaluation results. As FIG. 11 shows, a part of displacement measurement result 40 indicating the displacement information described referring to FIG. 8, and a part of evaluation result 39 of the elasticity information, for example, the shear wave velocity with accuracy (+/-7%), and Young's modulus together with the image data 26 indicating the subject and the evaluation position of the elasticity information. The accuracy may be the statistics such as the standard deviation of the results of measurements which have been conducted a plurality of times through repetitively executed sequence at the same position and the same angle as shown in FIG. 7. It is calculated through the generally employed evaluation method. The Young's modulus in a part of the evaluation result 39 of the elasticity information is derived as the elasticity information from the shear wave velocity by calculating the formula of E=3ρVs² (E: Young's modulus, p: density, Vs: shear wave velocity).

The probe used in the first embodiment has been described in a limited way to the convex type. However, it is essential for this embodiment to form the predetermined angle in accordance with the angle information between the shear wave propagation direction and the track pulse transmission-reception direction. Accordingly, the probe of arbitrary type may be employed without especially limiting. For example, it is possible to evaluate elasticity in the same process step with the same structure by changing the track pulse transmission-reception direction to the predetermined angular direction in accordance with the angle information under electronic control irrespective of the linear type probe in use.

The apparatus or the method as described in the first embodiment is expected to lessen the influence of the wave surface disturbance by reducing the propagation distance of the wave surface required to measure the shear wave velocity. The unified measurement position is also expected to improve the time resolution. Furthermore it is possible to establish the shear wave velocity measurement method, ensuring both measurement accuracy and reproducibility, thus realizing the ultrasonic diagnostic apparatus with high diagnosability.

### [Second Embodiment]

The ultrasonic diagnostic apparatus and the elasticity evaluation method according to a second embodiment will be described. The apparatus according to this embodiment includes the probe 11 which transmits and receives ultrasonic waves to and from the subject, and the controller 12 which transmits the first ultrasonic wave to the subject via the probe 11 to generate the shear wave in the subject, and transmits and receives the second ultrasonic wave to and from the subject. The controller 12 includes the speed measurement section 22 that calculates feature points 41, 42 indicating a plurality of divided regions based on the measurement position and the arrival time for the respective displacement data derived from the second ultrasonic wave in the regions divided in the subject based on the second ultrasonic wave, measures the propagation velocity of the shear wave based on the difference between the first arrival time and the second arrival time of the generated shear wave, which have been measured using the feature points indicating a plurality of calculated divided regions.

This embodiment provides the method of evaluating elasticity of the subject by transmitting the first ultrasonic wave to the subject from the probe to generate the sear wave in a plurality of divided regions in the subject, transmitting and receiving the second ultrasonic wave to and from the subject to calculate feature points indicating the divided regions for the respective displacement data of divided regions derived from the second ultrasonic wave based on the measurement position and the arrival time, and calculating the shear wave propagation velocity using the feature points indicating those divided regions.

The embodiment is featured by capability of lessening the influence of scattering on the shear wave surface resulting from propagation. In this embodiment, the center of gravity or the like is used as the feature point indicating the region or feature amount that captures the shear wave surface for the purpose of lessening the influence of scattering on the wave surface. The embodiment is intended to evaluate elasticity by dividing the inspection object of the subject into a plurality of regions, calculating the feature point indicating the divided region using the displacement data derived from the track pulse as the second ultrasonic wave in the respective regions, and calculating the shear wave propagation velocity based on the difference in the arrival time between the feature points indicating the divided regions.

In this embodiment, explanations of a series of the process from transmission of the push pulse as the first ultrasonic wave to the displacement measurement will be omitted as they are the same as those described in the first embodiment in reference to the general structure of the probe and the ultrasonic diagnostic apparatus shown in FIG. 1, the respective functional structures and information flow shown in FIG. 2, and steps 1 to 6 of the flowchart shown in FIG. 4.

FIG. 12 shows an example of the displacement data and displacement information used in the second embodiment. The drawing graphically represents an example that each center of gravity of the respective divided regions is calculated as the feature point indicating the divided region or the feature amount for capturing the wave surface, and the arrival time is measured by means of the calculated center of gravity. In this embodiment, the speed measurement is conducted by extracting the displacement data only at the positive or the negative side from those of the displacement measurement results as shown in FIG. 12, and the resultant two-dimensional wave surface is regarded as the rigid body. In other words, the displacement is replaced with the weight, and the center of gravity of the wave surface is focused to allow stable measurement of the arrival time irrespective of the disturbed wave surface, thus lessening the influence of scattering on the shear wave surface resulting from propagation. Extraction of the displacement at the positive side as shown in FIG. 12 will be described hereinafter.

FIG. 13 shows an example of the process steps according to the embodiment. In step 21, the inspection object of the subject is divided into regions. The number of divided regions is not specifically limited. It is the simplest way to divide the inspection object into two parts, that is, upper and lower regions with respect to the measurement position as the center (z=z₀) as shown in FIG. 12. In step 22, the maximum displacement value and arrival time at each depth are calculated in all the regions (z₁-z₂) used for the measurement. In step 23, the center of gravities 41 and 42 are calculated as the feature points indicating the upper wave surface and the lower wave surface, which have been divided, using the maximum displacement and the arrival time measured in step 22. Each of the center of gravities 41 and 42 as the example of the feature point indicating the region is obtained by calculating the numerical formula 5 shown in FIG. 6. Referring to the numerical formula 5, the codes d, u_{d}, t_{d} denote the depth, maximum displacement and arrival time, respectively.

FIG. 14 graphically represents calculation of the center of gravity as the feature amount of the wave surface captured as the rigid body, that is, the feature point indicating the region. Each set of the maximum displacement 44 (u_{d}) and the arrival time t_{d} is measured at the respective depths of n points from the depth d₁ to dₙ. The measured results are substituted for the numerical formula 5 shown in FIG. 6 to provide positions of the center of gravity. After the center of gravities 41 and 42 are obtained for the respective regions, the approximate straight line 43 for connecting the center of gravities 41 and 42 is calculated through the known fitting method such as the least-squares method in step 24. Values of the arrival time (t₁, t₂) are calculated from the intersection of the approximate straight line 43 and values of the depths (z₁, z₂) in step 25.

The embodiment configured to use the center of gravity as the feature point indicating the region allows calculation of the arrival time while comprehensively capturing the wave surface as a whole even if the wave surface is disturbed under the influence of scattering that occurs in the shear wave propagation process or the displacement locally disappears. This makes it possible to improve the measurement accuracy.

The elasticity evaluation method according to this embodiment considers the magnitude of displacement in calculation of the arrival time, and provides the calculated value resulting from weighting the highly sensitive displacement measurement result with high reliability. The inspection object is divided into three or more regions rather than two regions so that several results with higher values are only subjected to the fitting process so as to ensure the measurement with higher accuracy.

After the arrival time is calculated using the center of gravity of the divided region through the aforementioned process, the shear wave velocity is calculated in the similar way to the one described in the first embodiment. The elasticity information derived from the elasticity evaluation is sent to the display section 15, and presented to the operator.

FIG. 15 shows a display example according to the embodiment. The display screen of the display section 15 includes a part of image data 26 for indicating the tissue shape and the measurement position, a part of displacement measurement result 45 representing the displacement data of shear wave and the center of gravity shown in FIG. 12, and a part of evaluation result 46 indicating numerical values of the shear wave velocity and the like. The analytical method may be corrected on the screen. For example, the divided region for calculating the center of gravity is corrected by inputting the numerical value through the external input section 18 such as the keyboard as shown in FIG. 2 equipped with the apparatus. This makes it possible to immediately reflect the operation to the shear wave measurement result and the evaluation result. Likewise the first embodiment, if the wave transmission sequence is repeated a plurality of times as shown in FIG. 7 to execute plural measurements at the same measurement position, the statistics such as the standard deviation (+/- 7%) as the resultant accuracy will be displayed in the part of evaluation result. The error between the sample point and the approximate straight line is calculated in the fitting process so that the degree of the wave surface disturbance may be quantitatively evaluated. It is therefore effective to provide the index indicating reliability of the measurement and display numerical values for carrying out the highly accurate measurement. It is also effective to use the value of center of gravity in the fitting process as the index indicating reliability.

FIG. 16 is an explanatory view showing the effect derived from using the center of gravity with the configuration and the method according to this embodiment. Likewise FIG. 12, this drawing graphically represents the displacement that changes with time with respect to the distance in the depth direction and the depth. If the shear wave arrives parallel to the path of the track pulse as the second ultrasonic wave, the arrival time becomes the same at all the depths. However, as the upper part of FIG. 16 shows, if the wave surface disturbance is caused by scattering, the measurement result of the arrival time at each depth may have the error, and depending on the depth, the wave surface disappears, thus failing to measure the arrival time. On the contrary, as the lower part of FIG. 16 shows, in this embodiment, the wave surface is comprehensively captured as a single region enclosed with a broken line 47 so as to realize the measurement which lessens the influence of the wave surface disturbance. If the depth at which the wave surface disappears exists, the resultant displacement (mass of the rigid body) is sufficiently low compared to the surrounding regions, which hardly influences the position of the center of gravity as the feature point indicating the region used for the embodiment.

As for reliability shown in the part of evaluation result 46 of FIG. 15, by preliminarily storing reference values which have been measured, for example, the wave transmission condition, displacement corresponding to rigidity of the medium and values of the center of gravity in the memory in the controller 12 of the main body of the apparatus by means of gel phantom, it is possible to evaluate deviation of the measurement result from the ideal value in accordance with the ratio between the values and those measurement result.

In the aforementioned second embodiment, the probe of convex type is used in a limited way. However, it is essential to capture the wave surface as the single rigid body so as to measure the position of the center of gravity as the feature amount of the wave surface measurement. The probe to be used, therefore, is not specifically limited. For example, the linear type probe may be used for evaluating elasticity in the same process steps performed by the same apparatus configuration so long as the transmission-reception direction of the track pulse may be electronically controlled to a predetermined angular direction.

If a plurality of displacement measurement positions are set in the shear wave propagation direction, the method using the center of gravity as described in the embodiment is applicable. When setting the plurality of measurement positions, the number of displacement measurement results corresponds to that of the measurement positions. The center of gravity with respect to each result is calculated as the feature amount that captures the wave surface or the feature point indicating the region so as to calculate the arrival time of the wave surface at the respective measurement positions. Accordingly the shear wave velocity may be measured.

In the second embodiment as aforementioned, the center of gravity is used as the feature amount for capturing the wave surface or the feature point indicating the region. For example, arbitrary properties may be used as the feature amount, for example, minimum value, maximum value, intermediate value, average value of the wave surface, the inflexion point derived from the second order differentiation so long as the wave surface position is uniquely determined, thus allowing the process using the feature amount for capturing the wave surface, and the feature point indicating the region. This embodiment provides the ultrasonic diagnostic apparatus and the elasticity evaluation method which are capable of lessening the influence of the scattering on the shear wave surface resulting from the propagation.

The present invention is not limited to the embodiments as described above, and may include various modifications. The embodiments have been described in detail for better understanding of the invention, and are not necessarily restricted to the one provided with all the structures as described above. The structure of any one of the embodiments may be partially replaced with that of the other embodiment. Alternatively, it is possible to add the structure of any one of the embodiments to that of the other one. It is also possible to have the part of the structure of the respective embodiments added to, removed from and replaced with the other structure. The aforementioned structures, functions and processing sections may be realized by creating the program for partial or total execution, and the functions are realized by executing the program. They may be partially or totally implemented by the hardware, for example, designed with the integrated circuit.

### Reference Signs List

- 7: processing unit (CPU)
- 8: storage unit (memory)
- 9: control unit
- 10: ultrasonic diagnostic apparatus
- 11: probe
- 12: control unit
- 13: ultrasonic transmission-reception section
- 14: data processing section
- 15: display section
- 16: image data generation section
- 17: elasticity evaluation section
- 18: external input section
- 19: burst wave control section
- 20: pulse wave control section
- 21: displacement measurement section
- 22: speed measurement section
- 23: rectangular coordinate system
- 24: subject
- 25: pointer
- 26: image data
- 27: evaluation position
- 28: push position
- 29: track pulse transmission direction
- 30: shear wave
- 31: trigger signal
- 32: push pulse
- 33: track pulse
- 34: displacement at positive side
- 35: displacement at negative side
- 36: displacement measurement result at depth of z₁
- 37: displacement measurement result at depth of z₂
- 38, 43: approximate straight line
- 39, 46: evaluation result
- 40, 45: displacement measurement result
- 41, 42: center of gravity
- 44: maximum displacement at depth d
- 47: region where the center of gravity is measured

## Claims

1. An ultrasonic diagnostic apparatus comprising:
a probe which transmits and receives ultrasonic waves to and from a subject; and
a control unit which generates a shear wave in the subject by transmitting a first ultrasonic wave to the subject via the probe, and allows transmission and reception of a second ultrasonic wave to and from the subject, wherein the control unit includes a speed measurement section for measuring a first arrival time and a second arrival time of the shear wave generated in the subject based on the second ultrasonic wave, and measuring propagation velocity of the shear wave based on a difference between the first arrival time and the second arrival time.

2. The ultrasonic diagnostic apparatus according to claim 1, wherein the control unit makes a depth in the subject at which the first arrival time is measured different from a depth in the subject at which the second arrival time is measured.

3. The ultrasonic diagnostic apparatus according to claim 2, wherein the control unit makes the depth in the subject at which the second arrival time is measured larger than the depth in the subject at which the first arrival time is measured.

4. The ultrasonic diagnostic apparatus according to claim 3, wherein the control unit makes a transmission direction of the second ultrasonic wave different from a transmission direction of the first ultrasonic wave.

5. The ultrasonic diagnostic apparatus according to claim 4, wherein the control unit applies the second ultrasonic wave at a predetermined angle θ(θ≠0) toward a depth direction of the subject.

6. The ultrasonic diagnostic apparatus according to claim 1, wherein the control unit uses a burst wave as the first ultrasonic wave, and a track pulse formed of a plurality of pulse groups as the second ultrasonic wave.

7. The ultrasonic diagnostic apparatus according to claim 1, further comprising a display section for displaying image data of the subject or the propagation velocity.

8. The ultrasonic diagnostic apparatus according to claim 7,
wherein the control unit repeatedly executes transmission of the first ultrasonic wave to the subject, and transmission-reception of the second ultrasonic wave to and from the subject to calculate statistics of the propagation velocity of resultant values of a plurality of shear waves, and
the display section displays the statistics.

9. The ultrasonic diagnostic apparatus according to claim 7, wherein the control unit divides the inside of the subject into a plurality of divided regions, and calculates a feature point indicating each of the divided regions based on a measurement position and the arrival time with respect to displacement data derived from the second ultrasonic wave in the divided regions, and further measures the first arrival time and the second arrival time using the feature point indicating the region.

10. The ultrasonic diagnostic apparatus according to claim 9,
wherein the control unit uses the center of gravity of the divided region as the feature point indicating the region; and
the display section displays the displacement data and the center of gravity.

11. An elasticity evaluation method for evaluating elasticity of a subject by generating a shear wave in the subject to which a first ultrasonic wave is transmitted from a probe, transmitting and receiving a second ultrasonic wave to and from the subject, measuring a first arrival time and a second arrival time of the shear wave generated in the subject based on the second ultrasonic wave, and measuring a propagation velocity of the shear wave based on a difference between the first arrival time and the second arrival time.

12. The elasticity evaluation method according to claim 11, wherein the second ultrasonic wave is applied at a predetermined angle θ(θ≠0) toward a depth direction of the subject, and the first arrival time and the second arrival time are measured at different depths in the subject.

13. The elasticity evaluation method according to claim 11, wherein transmission of the first ultrasonic wave to the subject and transmission-reception of the second ultrasonic wave to and from the subject are repeatedly executed to calculate statistics of propagation velocity values of a plurality of generated shear waves.

14. The elasticity evaluation method according to claim 11, wherein the shear wave is generated in a plurality of divided regions in the subject, a feature point indicating the divided region is calculated for the respective displacement data of the divided regions, and the shear wave propagation velocity is calculated using the calculated feature points indicating a plurality of the divided regions.

15. The elasticity evaluation method according to claim 14, wherein the center of gravity of the divided region is used as the feature point indicating the divided region.
